(19) Europäisches Patentamt
European Patent Office
Office européen des brevets

(11) Veröffentlichungsnummer: **0 445 675 A2**

# (12) EUROPÄISCHE PATENTANMELDUNG

(21) Anmeldenummer: **91103141.7**

(51) Int. Cl.5: **G01N 35/08**, C12M 1/36

(22) Anmeldetag: **02.03.91**

(30) Priorität: **08.03.90 DE 4007246**

(43) Veröffentlichungstag der Anmeldung:
**11.09.91 Patentblatt 91/37**

(84) Benannte Vertragsstaaten:
**AT CH DE DK FR GB IT LI NL SE**

(71) Anmelder: **Forschungszentrum Jülich GmbH**
**Postfach 1913, Wilhelm-Johnen-Strasse**
**W-5170 Jülich(DE)**

(72) Erfinder: **Spohn, Uwe, Dr.**
**Kiefernweg 26**
**O-4050 Halle(DE)**
Erfinder: **Joksch, Burkhard**
**Münchener Strasse 5**
**W-5170 Jülich(DE)**
Erfinder: **Wandrey, Christian, Prof. Dr.**
**Wolfshovener Strasse 139**
**W-5170 Jülich(DE)**

## (54) Durchfluss-Analysenverfahren und -vorrichtung.

(57) Bei einem Durchfluß-Analysenverfahren zur Gehaltsbestimmung von Proben (FIA), die in einen Trägerstrom eingebracht und mit Reaktanten umgesetzt werden, der ein nachweisrelevantes Umsetzungsprodukt einem Detektor zuführt, werden Probe und Reaktant (vorzugsweise zeitlich aufeinander abgestimmt) zwei kontrollierten zeitprogrammierten Trägerströmen zugeführt, die über eine für die zu bestimmende Komponente und/oder den bzw. die Reaktanten semipermeable Trennschicht zusammengebracht werden. Der das Umsetzungsprodukt enthaltende Trägerstrom wird nachfolgend dem Detektor zugeführt. Als Trennschicht dient insbesondere eine poröse Dialysemembran von 10 - 25 $\mu$m Dicke mit selektiver Porengröße im Bereich zwischen 0,02 und 10 $\mu$m. Als Kontaktierungszelle, in der Proben- und Reagenzstrom über eine Membran in Kontakt gebracht werden, dienen insbesondere eine Dialysezelle oder Gasdiffusionszelle. Die neue Technik zeichnet sich durch hohe Selektivität und Unempfindlichkeit gegenüber Probenmatrixeinflüssen aus.

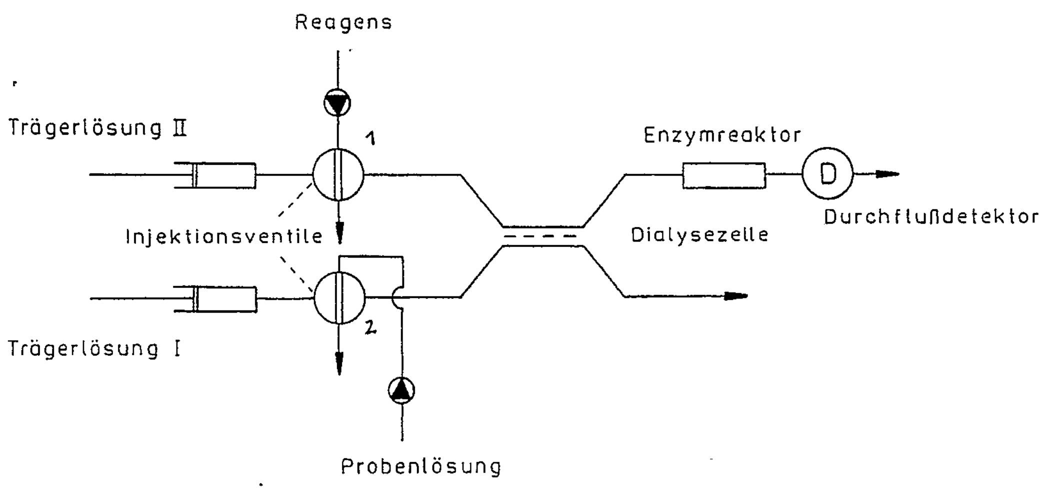


Fig. 1

EP 0 445 675 A2

Die Erfindung bezieht sich auf ein Durchfluß-Analysenverfahren zur Gehaltsbestimmung von Proben, die wiederkehrend in einen Trägerstrom eingebracht und nach Umsetzung mit zumindest einem Reaktanten einem Detektor zugeführt werden sowie auf eine dafür geeignete Anordnung.

Die Erfindung bezieht sich insbesondere auf die sog. Flow-Injection-Analyser Technik (FIA-Technik) und deren Anwendung zur Prozeßsteuerung.

Bei bekannten Durchfluß-Analysenverfahren werden Probenlösung und/oder Reagenzlösung einem Trägerstrom injiziert, der ggf. nach Passieren einer weiteren Reaktionsstrecke zum Detektor gelangt. Es ist auch bekannt, Probelösung und Reagenzlösung gesondert in getrennte Trägerströme einzubringen, die dann vermischt und nachfolgend dem Detektor zugeleitet werden. Im Falle, daß die Probelösung erhebliche Begleitkomponenten aufweist, wie z. B. Proteine oder dergleichen, die zu Störungen führen können, ist es auch bereits bekannt, solche Komponenten durch Dialyse zu entfernen. Dabei wird die Dialyse-Membran häufig kontinuierlich mit einem Probestrom belastet, so daß relativ häufig die Notwendigkeit zum Austausch bzw. zur Regenerierung besteht.

Ferner wird oft eine Verbesserung der Selektivität des Nachweises gewünscht sowie eine möglichst weitgehende Verminderung teurer Reagenzzugaben und Probensubstanzmengen.

Es wurde daher eine neue Technik entwickelt, mit der der Substanzverbrauch minimiert, die Selektion möglichst weit getrieben und die Analysenzeiten klein gehalten werden können.

Das zu diesem Zweck entwickelte erfindungsgemäße Verfahren der eingangs genannten Art ist dadurch gekennzeichnet,

daß man einen separaten Trägerstrom II für den oder die Reaktant(en) vorsieht, der über eine für die zu bestimmende(n) Komponente(n) und/oder den oder die Reaktant(en) semipermeable Trennschicht mit dem probenhaltigen Trägerstrom I in Kontakt gebracht wird, wobei die Fördergeschwindigkeit der Trägerströme I und II und die jeweilige Proben- und Reaktantenzugabe derart zeitlich programmiert gesteuert werden, daß sich die Konzentrations-Zeit-Profile der Bestimmungspartner in den Trägerlösungen an der Trennschicht je nach gewünscht er Empfindlichkeit zeitlich ganz oder teilweise überlappen und der das Umsetzungsprodukt enthaltende Trägerstrom zur Gehaltsbestimmung dem Detektor zugeführt wird.

Eine dafür geeignete Anordnung umfaßt im wesentlichen zumindest 2 separate Leitungszüge mit abgestimmter Förderung für Trägerströme, insbesondere Trägerlösungen, jeweils mit aufeinander abgestimmter gepulster Proben- bzw. Reaktanteninjektion; eine durch eine für zumindest einen der Bestimmungspartner semipermeable Trennschicht geteilte Kontaktierungszelle, zu deren unterschiedlichen Seiten die Leitungszüge führen und einen mit dem Ausgang der Zelle in Verbindung stehenden Detektor.

D. h., erfindungsgemäß erfolgt die Vermischung der Reaktionspartner unmittelbar an der Trennschicht in der Kontaktierungszelle, der zeitlich aufeinander abgestimmt begrenzte Proben- und Reaktantenmengen über die gesonderten Trägerströme zugeführt werden, wobei ggf. durch entsprechende Peakverschiebung eine Empfindlichkeitsminderung oder ggf. durch akkumulierte Aufnahme einer nur in geringer Menge vorhandenen Probe durch einen breiteren Reaktantenpeak oder ggf. längere Verweilzeiten in der Kontaktierungszelle durch entsprechende Steuerung der Transportmechanismen der Trägerströme für eine gesteigerte Empfindlichkeit gesorgt werden kann. Eine entsprechend angemessene Präzision der Arbeitsweise der Transportmechanismen ist dabei natürlich wichtig.

Das auch als analytisches Durchflußgradientenverfahren zu bezeichnende Verfahren kann zur schnellen und selektiven Bestimmung von Komponenten einer Probenlösung benutzt werden und kennzeichnet sich dadurch aus, daß die Probenlösung und eine oder mehrere Reaganzlösungen in durch eine semipermeable Trennschicht voneinander separierte Trägerströme so eingebracht werden, daß definierte und vornehmlich zeitlich kurze Konzentrations-Zeit-Gradienten der jeweils vorliegenden Komponenten entstehen, wobei sich die jeweiligen Komponenten nach Durchtritt durch die Membran in Abhängigkeit von der Membranpermeabilität und der gegenseitigen Zuordnung der Konzentrations-zeit-punkte vermischen und die sich ergebenden Konzentrationsgradienten eines oder mehrerer Reaktionsprodukte zur Gehaltsbestimmung dem oder den Detektoren zugeführt werden.

Dabei werden die durch die Probenlösung und die Reagenzlösungen bewirkten Konzentrations-Zeit-Gradienten zeitlich aufeinander abgestimmt sychron oder asychron den bezüglich der Durchflußgeschwindigkeiten zeitlich gesteuerten Trägerströmen zugeführt. Insbesondere können die Konzentrations-Zeit-Profile als definierte lineare, exponentielle und andere arithmetrisch beschreibbare Funktionen der Konzentration von der Zeit eingestellt werden. Zweckmäßigerweise werden die Konzentrations-Zeit-Profile durch Anhalten und Wiederinbewegungsetzen der Trägerströme diskontinuierlich transportiert und für die sich ergebenden Misch- und Reaktionszonen unterschiedliche Reaktionszeiten eingestellt.

Die Konzentrations-Zeit-Profile der miteinander reagierenden bzw. sich miteinander vermischenden Komponenten erzeugen an der Trennschicht je nach einzustellender Empfindlichkeit ganz oder teilweise zeitlich überlappende und in Abhängigkeit von der Membranpermeabilität durchdringende und definierte

Reaktions- bzw. Mischzonen.

Als Trennschicht eignet sich insbesondere eine transportselektive Membran von < 1 mm Dicke wie z.B. eine homogene oder hydrophob mikroporöse Membran für die Permeation flüchtiger Substanzen, eine mikroporöse Dialysemembran für die Permeation niedermolekularer Substanzen, eine Ionenaustauschermembran für die Permeation von Kationen bzw. Anionen oder eine neutrale und immobilisierte Trägermoleküle enthaltende Trennmembran.

Vorzugsweise sind ein oder mehrere weitere Reaktionspartner in der Trennschicht immobilisiert und bewirken eine selektive Umwandlung einer oder mehrerer Komponenten der Probenlösung beim Vorbeiströmen oder beim Membrandurchtritt.

Für die Durchführung mehrerer Bestimmungen in der gleichen Probenlösung oder zur weiteren Selektivitätserhöhung können mehrere in Reihe oder parallel geschaltete Membrankontaktierungszellen für das jeweils anzuwendende Membrantrennverfahren oder die jeweils gewünschte chemische Konversion einer Proben- bzw. Reagenzkomponente vorgesehen werden. Zweckmäßigerweise werden schließlich Konzentrations-Zeit-Gradienten in Peak-, Dreiecks-und Rampenform für die Komponenten der Proben- und der Reagenzlösung erzeugt.

Besonderheiten der Erfindung gehen aus den Unteransprüchen hervor.

Nachfolgend wird die Erfindung anhand der beigefügten Zeichnungen erläutert, die schematisch spezielle Beispiele veranschaulichen. Es zeigen:

| | |
|---|---|
| Figur 1 | eine erste Ausführungsart der Erfindung für eine enzymatische Bestimmung von Probenkomponenten; |
| Figur 2a, 2b | die Überlappung der Konzentrations-Zeit-Profile von in definierter Form in die Trägerströme I und II eingebrachten Konzentrationsgradienten der interessierenden Probensubstanz - und des Reagenzes, C = Konzentration, $C_R^\circ$ = injizierte Konzentration des Reagenzes, $C_P^\circ$ = injizierte Konzentration der Probenlösung, $C_{h1}$ und $C_{h2}$ = maximale durch den Detektor zu messende Konzentration; U.ZW. |
| Figur 2a | bei synchroner Injektion von Proben- und Reagenzlösung in die in Figur 1 dargestellten Meßanordnung; |
| Figur 2b | bei asynchroner Injektion von Proben- und Reagenzlösung in die in Figur 1 dargestellten Meßanordnung; |
| Figur 3 | eine Variation der in Figur 1 gezeigten Anordnung mit zwei Kontaktierungszellen; |
| Figur 4 | eine zweite Ausführungsart der Erfindung für eine selektive durchflußtitrimetrische Bestimmung flüchtiger Probenkomponenten und |
| Figur 5 | die Überlappung des Probensubstanz-Konzentrations-Profiles mit dem dreieckförmigen Reagenz-Konzentrationsprofil; |
| Figuren 6 und 7 | zwei schematische Darstellungen, die den Einsatz des erfindungsgemäßen Prinzips zur Prozeßsteuerung wiedergeben. |

Gemäß Figur 1 umfaßt die erfindungsgemäße Anordnung zwei durch eine Dialysemembran getrennte Strömungskanäle, durch die eine als Donor und eine als Akzeptor dienende Trägerlösung gepumpt werden. Die Akzeptorlösung II fließt nach Passieren der Kontaktierungszelle über einen Enzymreaktor, ausgeführt in Form einer kleinen mit Enzymimmobilisat gepackten Säule, zu einem als Durchflußdetektor dienenden Fluoreszenzdetektor.

Zeitlich aufeinander abgestimmt werden über die Ventile 1 das Reagens $NAD^+$ und über Ventil 2 Isoleucin enthaltende Probenlösung injiziert. Auf den Wegen zwischen den Ventilen und der Kontaktierungszelle bilden sich in der Donor- und in der Akzeptorlösung reproduzierbare Konzentrations-Zeit-Profile aus, die sich an den gegenüberliegenden Seiten der Dialysemembran begegnen. In dieser Kontaktierungszelle gelangt aus der Probe stammendes Isoleucin durch die semipermeable Membran in das Konzentrations-Zeit-Profil des in der Trägerlösung II vorhandenen $NAD^+$. Vom Ausgang der Kontaktierungszelle gelangt die nunmehr Isoleucin und $NAD^+$ enthaltende Trägerlösung II durch den Enzymreaktor, der im wesentlichen durch ein gepacktes Strömungsrohr mit immobilisierter Leucindehydrogenase gebildet wird und in dem die Bestimmungsreaktion

$$\text{L - Isoleucin} + NAD^+ \rightarrow \alpha\text{- Ketoisoleucin} + NADH + H^+$$

abläuft, deren Reaktionsprodukt NADH durch einen Fluoreszenzdetektor gemessen wird. Die sich aufgrund des zwischen den Injektionsventilen und dem Detektor bestehenden konstanten Ort-Zeit-Regimes reproduzierbar ausbildenden Fluoreszenz-Zeit-Verläufe werden bezügl. der Peakfläche ausgewertet und sind mit der Isoleucinkonzentration korrelierbar.

Auf diese Weise werden die analytischen Operationen (Abtrennung aus der Probenmatrix, Diffusion der

Probensubstanz in das und Vermischung mit dem Reagenz) auf einem sehr kleinen Abschnitt der Strömungskanäle in sehr kurzer Zeit durchgeführt. Durch entsprechende Miniaturisierung der Kontaktierungszelle läßt sich die Verbreiterung der Konzentrations-Zeit-Profile im Vergleich zur getrennten Durchführung der genannten analytischen Operationen wesentlich verringern, wodurch sich eine größere Injektions- und damit Signalfrequenz realisieren läßt. Kürzere Analysenzeiten werden möglich.

Die beschriebene Anordnung zeichnet sich durch hohe Selektivität und Unempfindlichkeit gegenüber Probenmatrixeinflüssen aus. So werden bei der Penetration des Isoleucins aus der Probenlösung über die Dialysemembran in die Reagenzlösung alle höheren molekularen Substanzen, wie z.B. Proteasen und Proteine zurückgehalten.

Da über die Ventile 1 und 2 auch sehr kleine Lösungsvolumina von $< 10\ \mu l$ mit hoher Präszision injiziert werden können, resultiert ein sehr kleiner Verbrauch an Reagenz und ein sehr kleiner Probensubstanzeinsatz, verbunden mit einer hohen Analysengenauigkeit und -empfindlichkeit.

Generell können die separaten Trägerlösungen kontinuierlich oder nach einem bestimmten Zeitprogramm pneumatisch, hydrostatisch oder mit Hilfe von Schlauchpumpen peristaltisch bewegt werden. Besonders bevorzugt sind Präzisionskolbenpumpen. Als Trennschicht eignen sich poröse homogene oder heterogene Membranen, Flüssigmembranen oder dünne Luftschichten, insbesondere sehr dünne Dialysemembranen mit einem Cut-off von 5000 bis 50000 oder hydrophobe poröse Polytetrafluorethylen-Membranen.

Die Injektion erfolgt insbesondere durch Schleifeninjektionsventile, z.B. durch zwei getrennte und zeitlich unabhängig voneinander gesteuerte und elektromagnetisch oder pneumatisch gestellte Rotor- oder Schiebeventile, sie kann aber auch durch ein Mehrfachschleifenventil erfolgen.

Figuren 2a und 2b zeigen die Überlappung von Probensubstanz- und Reagenz-Konzentrations-Profil. Die schraffierte Fläche bestimmt die Empfindlichkeit der Bestimmung, da nur in diesem Bereich sowohl $NAD^+$ als Isoleucin vorliegen und die enzymatische Umsetzung möglich ist. Auf diese Weise werden bei zeitversetzter Injektion der Proben- und der Substanzlösung unterschiedliche effektive Verdünnungsfaktoren und damit Empfindlichkeiten über die Differenz der Injektionszeiten eingestellt.

Figur 3 zeigt eine Abwandlung der in Figur 1 gezeigten Ausführungsart, bei der über Dreiwegeventile umschaltbar zwei parallel vorgesehene Kontaktierungszellen angesteuert werden können, mit jeweiliger Trennschicht, die unterschiedliche Probenkomponenten passieren läßt: so können durch eine Gasdiffusionsschicht flüchtiges Material wie Ethanol oder Acetaldehyd abgetrennt und analysiert werden, während z.B. Glucose oder Aminosäuren durch eine hydrophile und mikroporöse Membran diffundieren können und in der Dialysezelle von der Probenlösung separiert werden.

Figur 4 zeigt die Anwendung der Erfindung zur selektiven Titration einer über eine semipermeable Membran abgetrennten flüchtigen Substanz mit einem aus zwei linearen Konzentrationsgradienten zusammengesetzten Konzentrationsprofil eines geeigneten Titrationsmittels, z.B. von Kohlendioxid mit Hydroxidionen. Die Probenlösung wird über ein Injektionsventil mit sehr großer Proben-Injektionsschleife in den Donorstrom der Gasdialysezelle injiziert. Das Konzentrationsprofil des Titrationsmittels wird durch Elektrolyse an einer miniaturisierten Elektrode durch ein dreieckförmiges Strom-Zeit-Profil erzeugt. Das Strom-Zeit-Profil wird durch einen Dreieckstromgenerator, verwirklicht durch eine computergesteuerte Spannungsquelle mit nachgeschaltetem Spannungsstromwandler, erzeugt. Der nachgeschaltete photometrische Durchflußdetektor zeichnet zwei sich gegenüberliegende Titrationskurven auf, wobei durch Bestimmung der zeitlichen Differenz zwischen den paarigen Äquivalenzpunkten die Berechnung der abgetrennten Kohlendioxidkonzentration ermöglicht wird. Wird das Titrationsreagenz als Konzentrations-Zeit-Profil in Form eines gleichschenkligen Dreiecks erzeugt bzw. dem Trägerstrom I zugeführt, resultieren zwei zueinander spiegelsymmetrische Titrationskurven. In der miniaturisierten Gasdiffusionszelle wird die zu bestimmende flüchtige Substanz abgetrennt, angereichert und (bei vorgeschalteter Elektrolyse-Zelle) titriert, wodurch eine bei Titrationen ungewöhnlich hohe Selektivität und sehr kurze Titrationszeiten erreicht werden. Das Analysenergebnis wird nach der Gleichung

$$C_S = b/a \frac{I_{max}}{F z V_T} \left(1 - \frac{\Delta t}{2\tau}\right)$$

berechnet, wobei $C_S$ die Probenkonzentration, $I_{max}$ die maximal durchlaufene Elektrolysestromstärke, F die Faraday-Konstante, z die Zahl der ausgetauschten Elektronen, $V_T$ die Volumendurchflußgeschwindigkeit der Probenlösung, f der Grad des Stoffüberganges der zu bestimmenden Substanz über die Trennmembran, $\Delta t$

die zeitliche Differenz zwischen den durchlaufenden Äquivalenzpunkten, 2 $\tau$ die Zeitdauer des Reagenz-Gradienten-Profil-Durchlaufes sowie a und b die Stöchiometriefaktoren der Reaktion zwischen Probensubstanz und Titrationsmittel (Reagenz) sind.

Figur 5 zeigt das Meß- und Auswertungsprinzip.

Figur 6 zeigt die Anwendung der Erfindung zur Prozeßsteuerung bei einer Isoleucin-Fermentation: den Trägerströmungen I und II werden programmiert über die Ventile 1 und 2 gepulste Proben bzw. Cofaktoren zugeführt. Die beiden Ströme gelangen dann zur Dialysezelle oder zur Gasdiffusionszelle, deren Ablauf über eine Art Ausgleichsstrecke programmiert zu einer Reihe von parallelgeschalteten Enzymreaktoren und dann in den Fluoreszenz-Detektor gelangt. Dessen jeweiliges Konzentrationssignal wird einem Prozeßrechner zugeführt, der daraufhin die Fermentation steuert.

Figur 7 zeigt als weiteres Anwendungsbeispiel den Einsatz der erfindungsgemäßen Anordnung zur Steuerung einer Ethanol-Fermentation. Die gezeigte Anordnung entspricht im Aufbau im wesentlichen der Anordnung gemäß Figur 3 und dürfte damit aus sich selbst heraus verständlich sein.

## Patentansprüche

1. Durchfluß-Analysenverfahren zur Gehaltsbestimmung von Proben, die wiederkehrend in einen Trägerstrom I eingebracht und nach Umsetzung mit zumindest einem Reaktanten einem Detektor zugeführt werden,
   **dadurch gekennzeichnet,**
   daß man einen separaten Trägerstrom II für den oder die Reaktant(en) vorsieht, der über eine für die zu bestimmende(n) Komponente(n) und/oder den oder die Reaktant(en) semipermeable Trennschicht mit dem probenhaltigen Trägerstrom I in Kontakt gebracht wird, wobei die Fördergeschwindigkeit der Trägerströme I und II und die jeweilige Proben- und Reaktantenzugabe derart zeitlich programmiert gesteuert werden, daß sich die Konzentrations-Zeit-Profile der Bestimmungspartner in den Trägerlösungen an der Trennschicht je nach gewünschter Empfindlichkeit zeitlich ganz oder teilweise überlappen und der das Umsetzungsprodukt enthaltende Trägerstrom zur Gehaltsbestimmung dem Detektor zugeführt wird.

2. Verfahren nach Anspruch 1,
   **dadurch gekennzeichnet,**
   daß als Trennschicht eine dünne semipermeable Membran von $\leqq$ 1 mm Dicke, insbesondere eine poröse Dialyse-Membran von 10 bis 25 $\mu$m Dicke mit selektiver Porengröße im Bereich zwischen 0,02 und 10 $\mu$m verwendet wird.

3. Verfahren nach Anspruch 1 oder 2,
   **dadurch gekennzeichnet,**
   daß ein weiterer Reaktionspartner in der Trennschicht vorgesehen wird.

4. Verfahren nach einem der vorangehenden Ansprüche,
   **dadurch gekennzeichnet,**
   daß für die Durchführung mehrerer Bestimmungen an ein und derselben Probe mehrere in Reihe oder parallel geschaltete mit semipermeabler Membran ausgestattete Kontaktierungszellen für die jeweilige Permeation von Bestimmungskomponenten vorgesehen werden.

5. Verfahren nach einem der vorangehenden Ansprüche,
   **dadurch gekennzeichnet,**
   daß die semipermeable Trennschicht für die zu bestimmende Probenkomponente durchlässig ist und daß das Konzentrationszeitprofil des Stimmungspartners im Trägerstrom II vor oder hinter der Trennschicht elektrolytisch erzeugt wird.

6. Verfahren nach einem der vorangehenden Ansprüche,
   **dadurch gekennzeichnet,**
   daß die Konzentrationszeitprofile der Bestimmungspartner in Dreiecks- bzw. Rampenform erzeugt werden.

7. Anordnung zur Durchführung des Verfahrens nach den Ansprüchen 1 bis 6,
   **gekennzeichnet durch**

zumindest zwei separate Leitungszüge mit abgestimmter Förderung für Trägerströme, insbesondere Trägerlösungen, jeweils mit aufeinander abgestimmter gepulster Proben- bzw. Reaktanteninjektion; eine durch eine für zumindest einen der Bestimmungspartner semipermeable Trennschicht geteilte Kontaktierungszelle, zu deren unterschiedlichen Seiten die Leitungszüge führen und durch einen mit dem Ausgang der Zelle in Verbindung stehenden Detektor.

8. Anordnung nach Anspruch 7,
**gekennzeichnet durch**
zumindest eine Dialysezelle oder Gasdiffusionszelle als Kontaktierungszelle.

9. Anordnung nach Anspruch 7 oder 8,
**dadurch gekennzeichnet,**
daß die Schichtdicke der Flüssigkeitsströme in der Kontaktierungszelle $\leq$ 1 mm ist.

10. Anordnung nach einem der Ansprüche 7 bis 9,
**gekennzeichnet durch**
jeweilige Fördermechanismen nach dem Präzisions-Verdrängerprinzip, insbesondere Präzisions-Kolbenpumpen.

11. Anordnung nach einem der Ansprüche 7 bis 10,
**gekennzeichnet durch**
einen Cofaktor- oder Enzym-Vorrat für die Reaktanten-Injektion.

12. Anordnung nach einem der Ansprüche 7 bis 11,
**gekennzeichnet durch**
einen Cofaktor-Vorrat für die Reaktanten-Injektion und zumindest einen Enzym-Vorrat zwischen Kontaktierungszelle und Detektor.

13. Anordnung nach einem der vorangehenden Ansprüche 7 bis 12, gekennzeichnet durch mehrere in Reihe oder parallel geschaltete Kontaktierungszellen.

14. Anwendung einer Anordnung nach einem der Ansprüche 7 bis 13 zur Prozeßsteuerung insbesondere für Fermentationsprozesse.

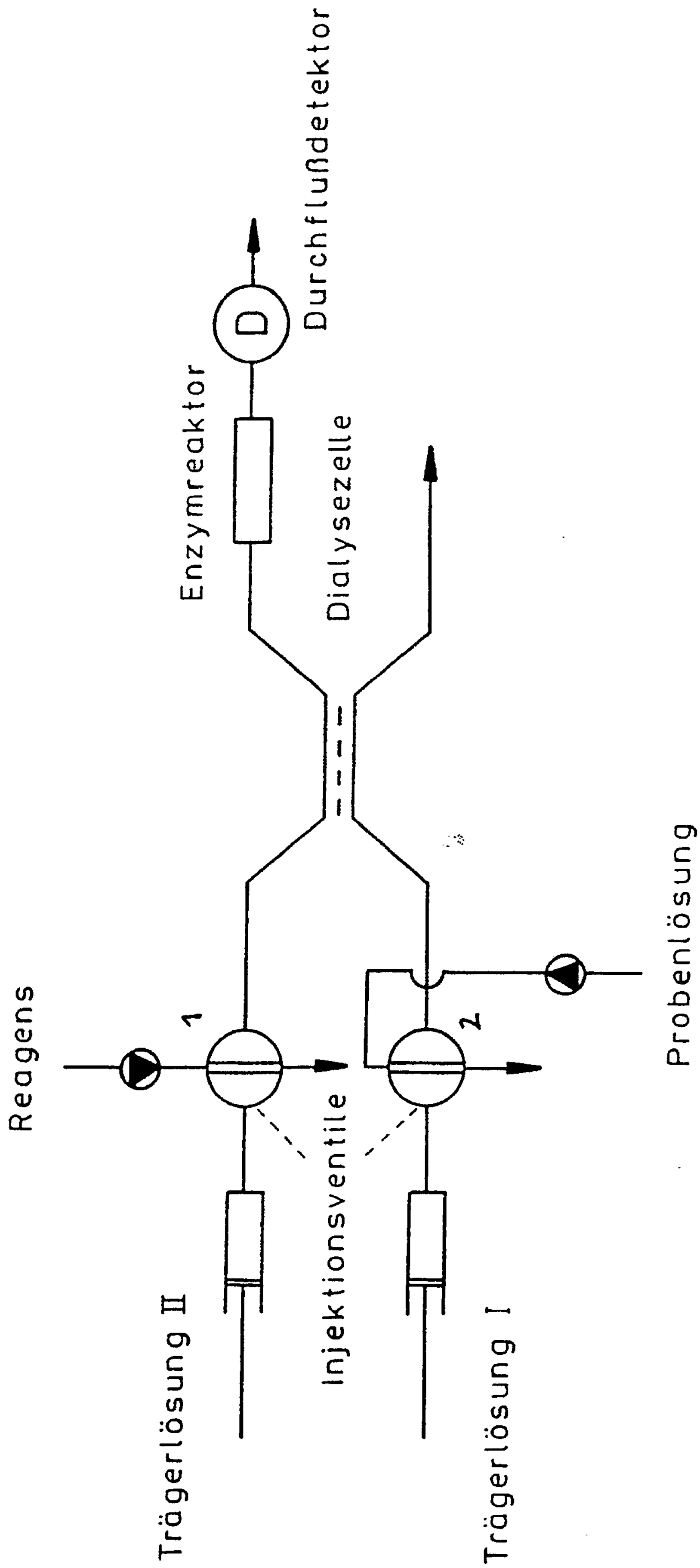
Durchflußdetektor
D
Enzymreaktor
Dialysezelle
Reagens
1
2
Probenlösung
Injektionsventile
Trägerlösung II
Trägerlösung I

Fig. 1

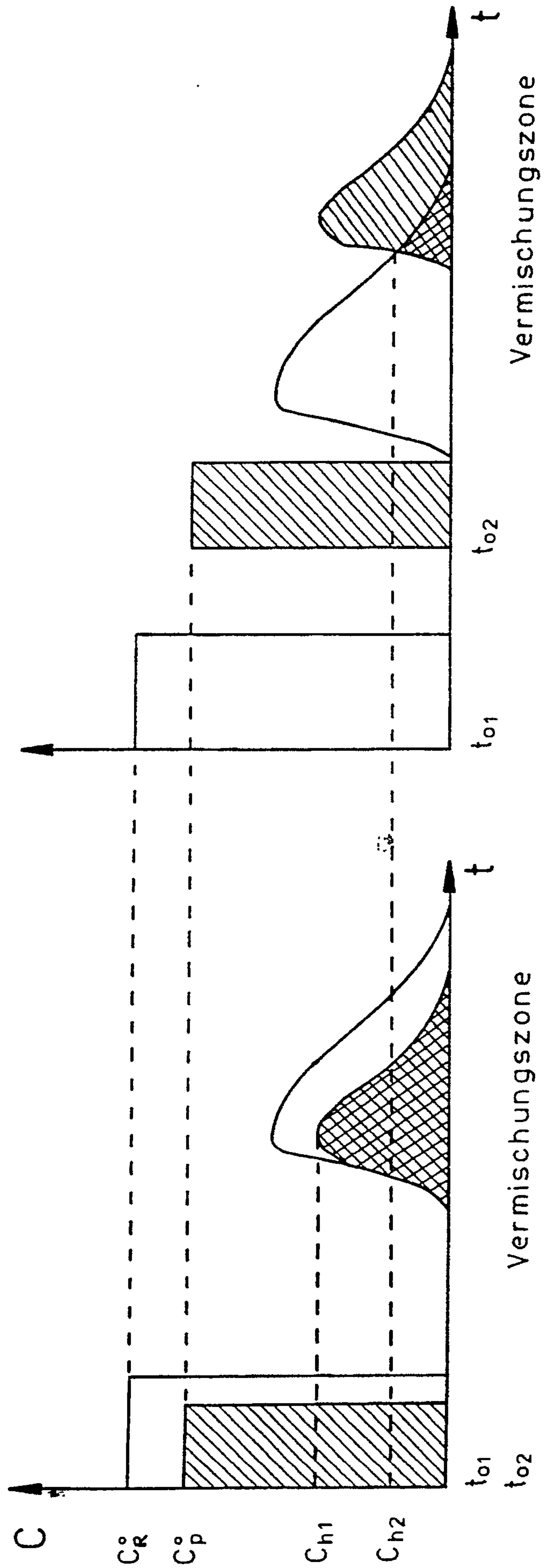
C
$C^\circ_R$
$C^\circ_p$
$C_{h1}$
$C_{h2}$
$t_{o1}$
$t_{o2}$
t
Vermischungszone
Vermischungszone

Fig. 2a

Fig. 2b

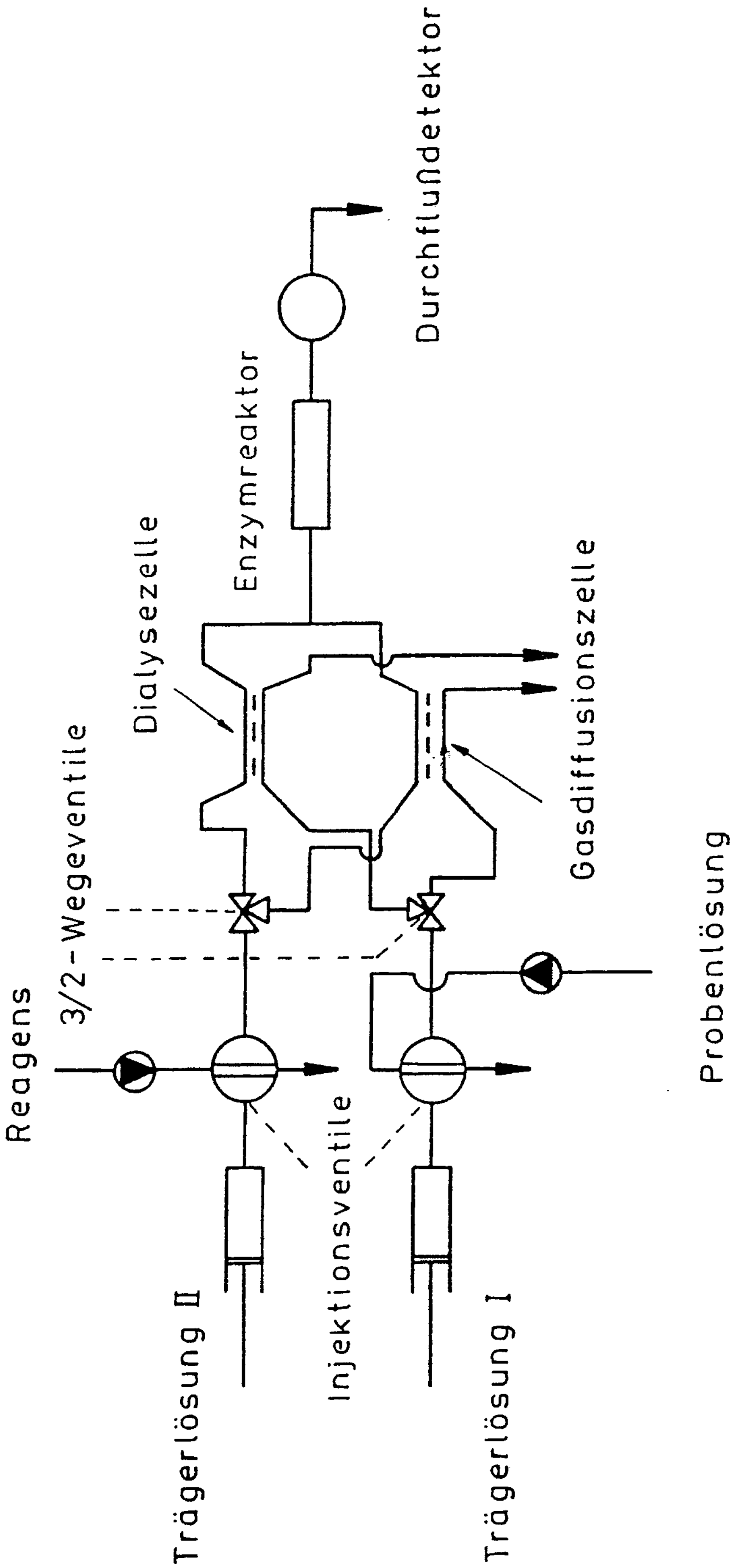
Durchflußdetektor
Enzymreaktor
Dialysezelle
Gasdiffusionszelle
3/2-Wegeventile
Reagens
Probenlösung
Injektionsventile
Trägerlösung II
Trägerlösung I

Fig. 3

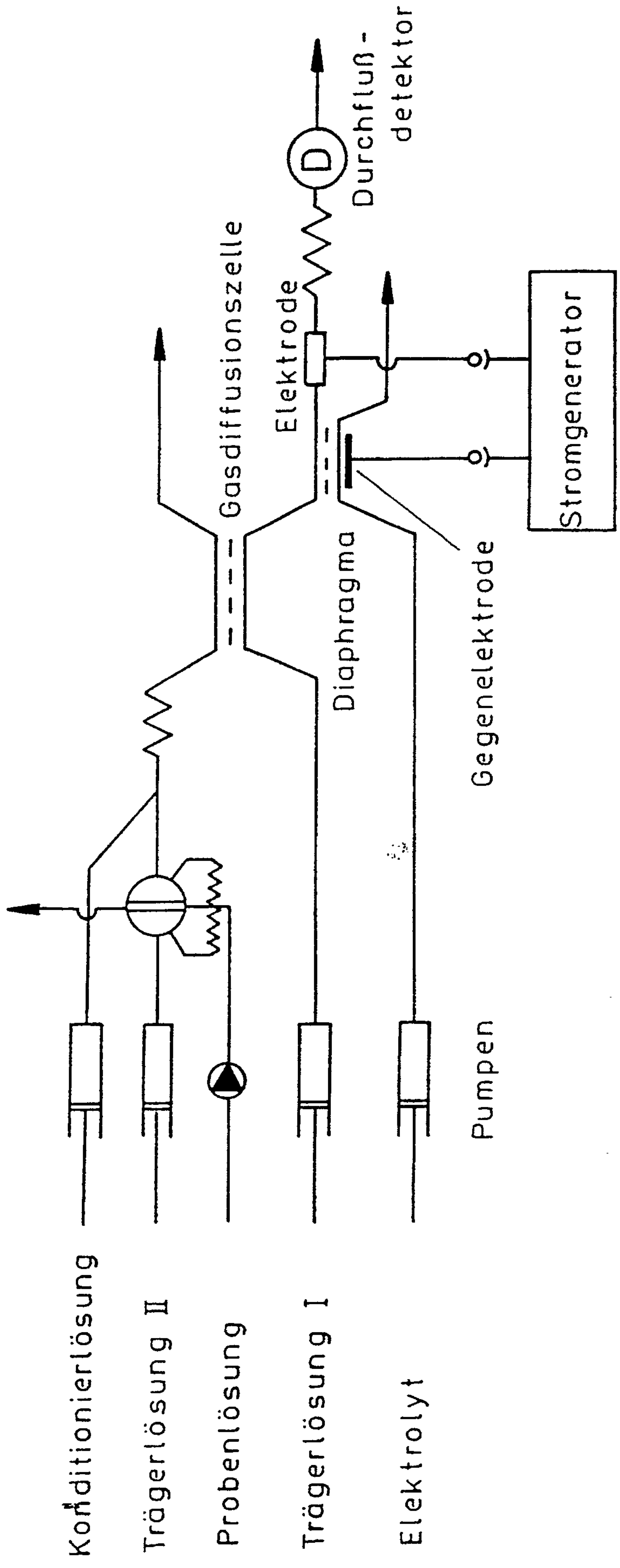
Konditionierlösung
Trägerlösung II
Probenlösung
Trägerlösung I
Elektrolyt
Pumpen
Gasdiffusionszelle
Elektrode
D
Durchfluß-
detektor
Diaphragma
Gegenelektrode
Stromgenerator

Fig. 4

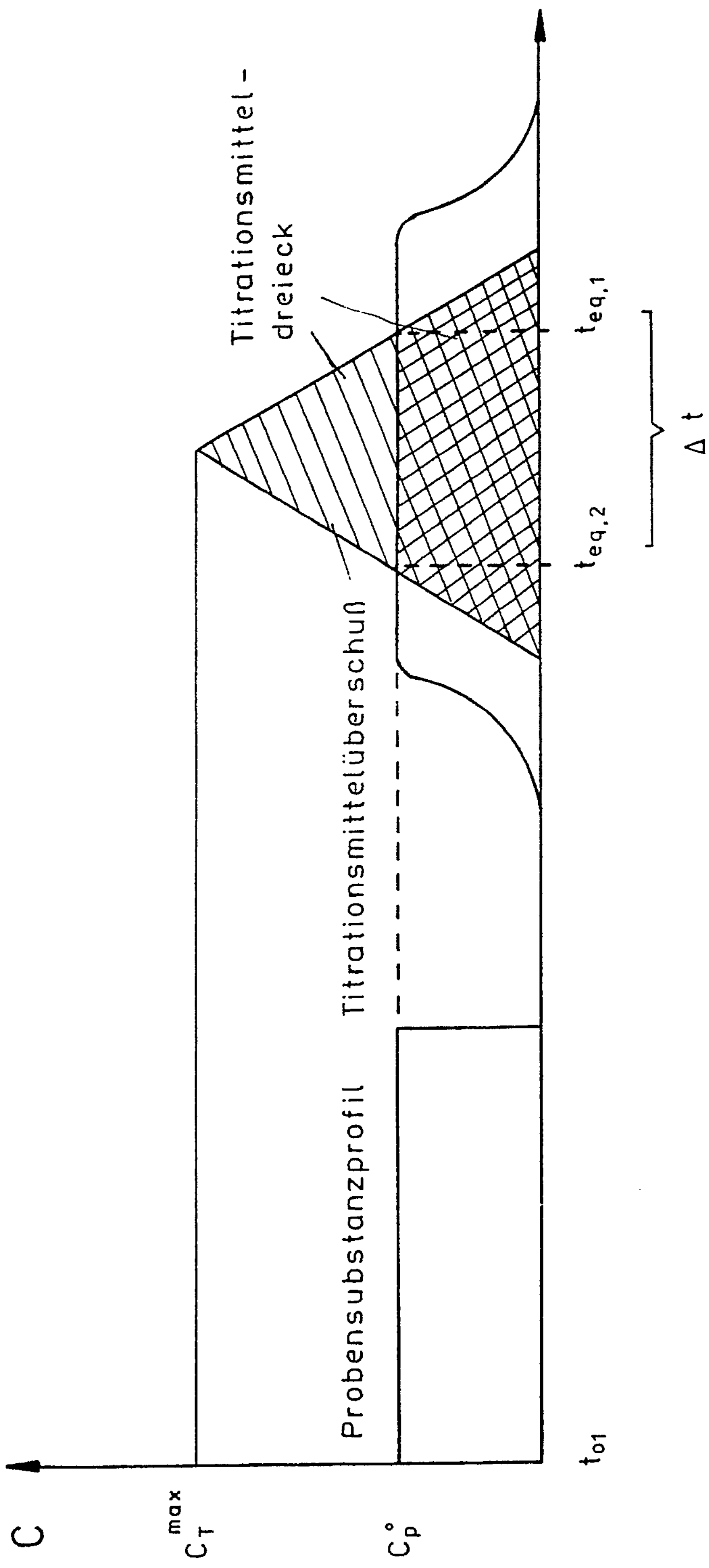
C
$C_T^{max}$
$C_p^{\circ}$
$t_{o1}$
Probensubstanzprofil
Titrationsmittelüberschuß
Titrationsmittel-
dreieck
$t_{eq,1}$
$t_{eq,2}$
$\Delta t$

Fig. 5

6 – Channel – FIA for Online – Monitoring of a bacterial Isoleucinfermentation (Example of application)

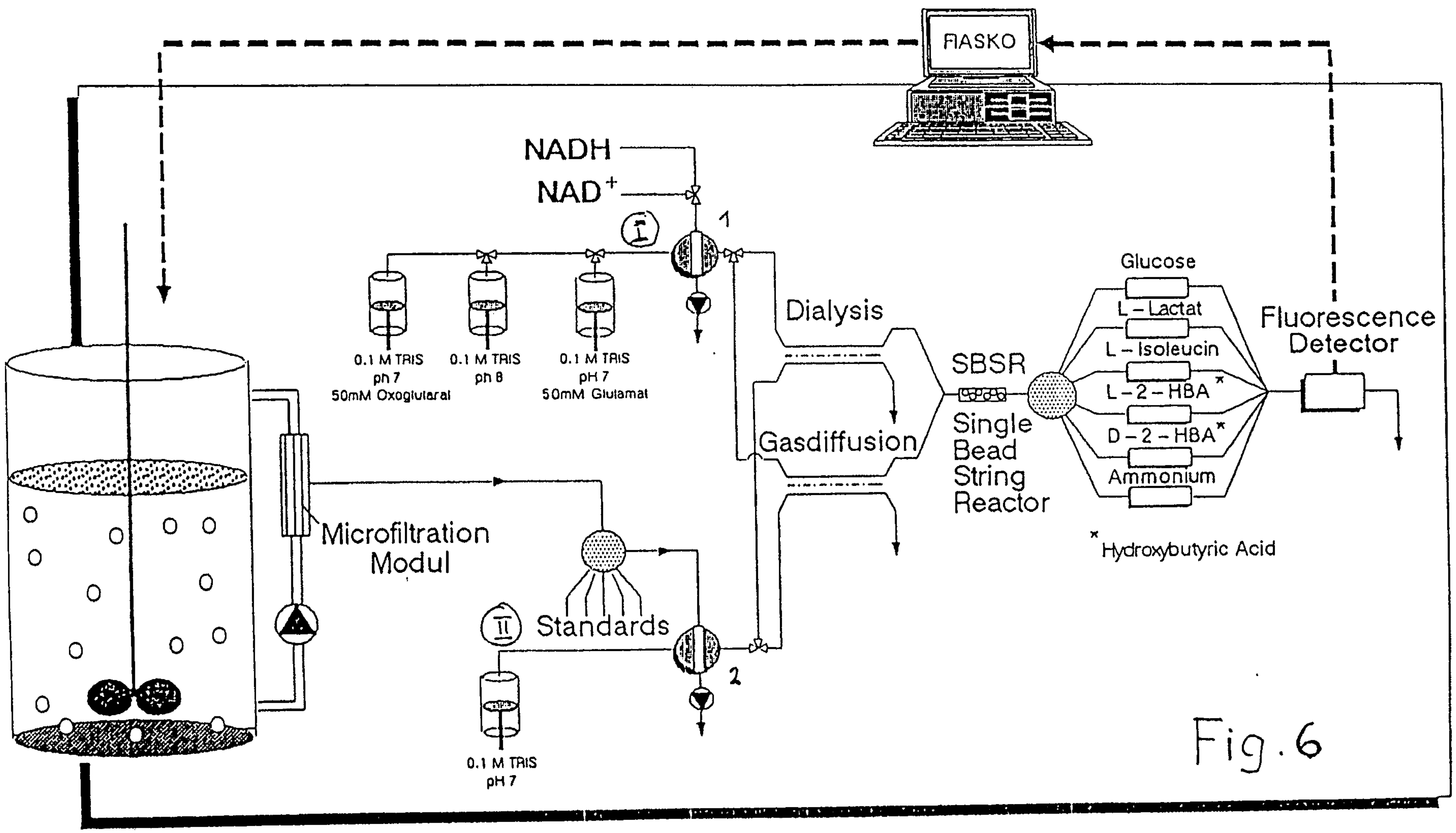


Fig. 6

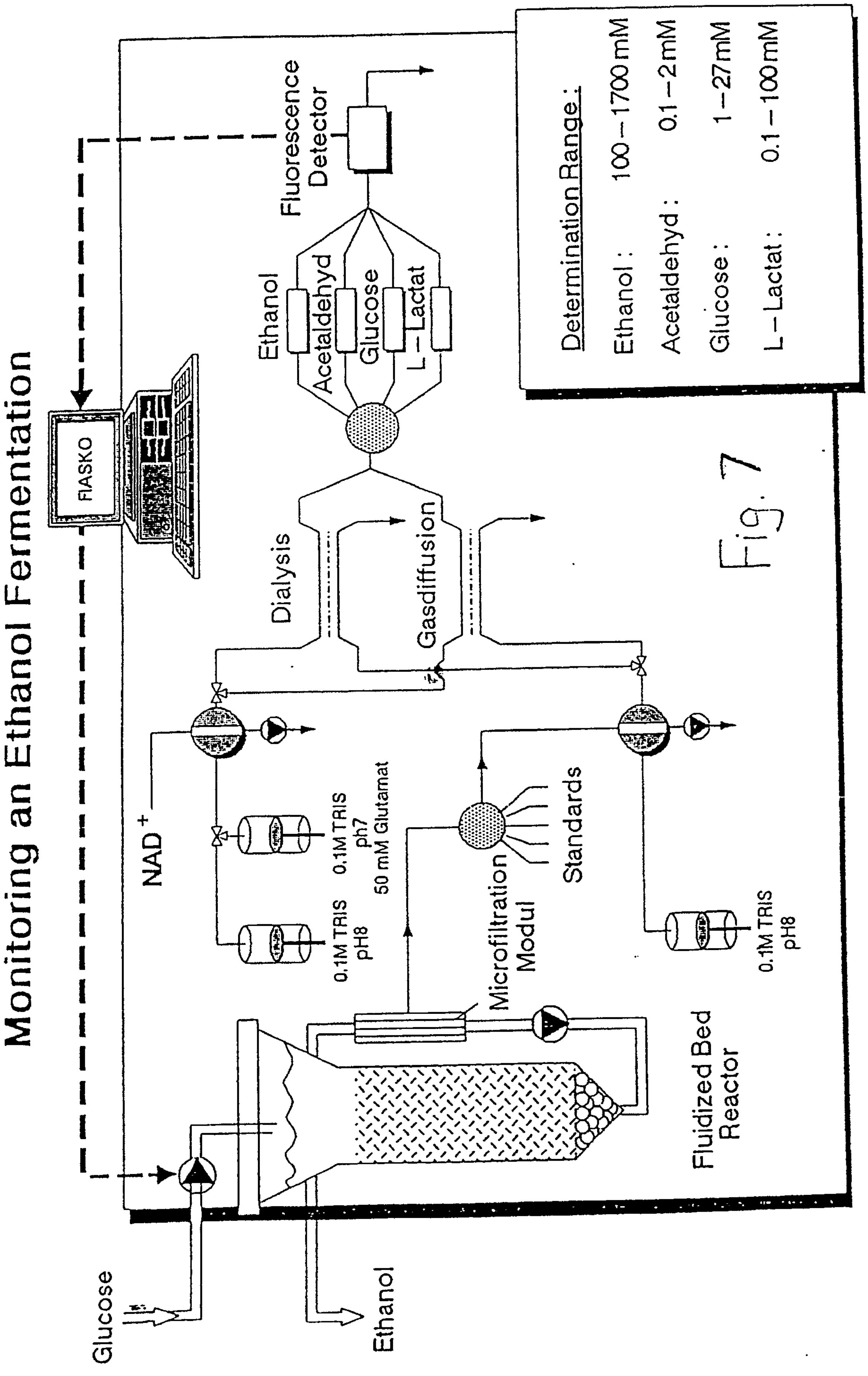
Monitoring an Ethanol Fermentation
FIASKO
Fluorescence Detector
Ethanol
Acetaldehyd
Glucose
L-Lactat
Dialysis
Gasdiffusion
NAD+
0.1M TRIS pH8
0.1M TRIS ph7 50 mM Glutamat
Microfiltration Modul
Standards
0.1M TRIS pH8
Fluidized Bed Reactor
Glucose
Ethanol
Determination Range :
Ethanol : 100 – 1700 mM
Acetaldehyd : 0.1 – 2 mM
Glucose : 1 – 27 mM
L-Lactat : 0.1 – 100 mM

Fig. 7